# EUROPEAN PATENT APPLICATION

(11) **EP 4 056 200 A1**
(43) Date of publication of application: **14.09.2022**
(21) Application number: 20886098.1
(22) Date of filing: 04.11.2020
(51) Int. Cl.: A61K 39/395, A61K 31/4709, C07K 16/28, A61P 35/00

(54) **DRUG COMBINATION OF QUINOLINE DERIVATIVE AND PD-1 MONOCLONAL ANTIBODY**

(30) Priority: 04.11.2019 CN 201911064102
(71) Applicant: CHIA TAI TIANQING PHARMACEUTICAL GROUP CO., LTD., Lianyungang, Jiangsu 222062 (CN); Innovent Biologics (Suzhou) Co., Ltd., Suzhou, Jiangsu 215123 (CN)
(72) Inventor: LI, Jundong, Guangzhou, Guangdong 510060 (CN); WEI, Wei, Guangzhou, Guangdong 510060 (CN); YANG, Fan, Guangzhou, Guangdong 510060 (CN); LIU, Zheng, Nanjing, Jiangsu 211100 (CN); CHEN, Zekun, Nanjing, Jiangsu 211100 (CN)
(74) Representative: v. Bezold & Partner Patentanwälte - PartG mbB
(86) International application number: PCT/CN2020/126422
(87) International publication number: WO 2021/088853

(57) **Abstract**

Provided is a drug combination of a quinoline derivative and a PD-1 monoclonal antibody, which contains a tyrosine kinase inhibitor and an immune checkpoint inhibitor, wherein the tyrosine kinase inhibitor is a compound represented by Formula I or a pharmaceutically acceptable salt thereof. The drug combination has good activity against endometrial tumors.

## Description

### TECHNICAL FIELD

The present application belongs to the field of medical technology, and relates to a combination therapy against tumor(s). Specifically, the present application relates to a combination based on quinoline derivatives and PD-1 monoclonal antibody and use thereof in anti-tumor.

### BACKGROUND

Tyrosine kinases are a group of enzymes that catalyze the phosphorylation of tyrosine residues of protein and play an important role in intracellular signal transduction. It is involved in the regulation, signal transmission and development of normal cells and is also closely related to the proliferation, differentiation, migration and apoptosis of tumor cells. Many receptor tyrosine kinases are related to the formation of tumors, and can be divided into epidermal growth factor receptor (EGFR), platelet-derived growth factor receptor (PDGFR), vascular endothelial growth factor receptor (VEGFR), fibroblast growth factor receptor (FGFR), etc. according to the structural differences of their extracellular regions.

Anlotinib is a quinoline derivative tyrosine kinase inhibitor, which acts as a multi-targeted tyrosine kinase inhibitor (TKI) in affecting tumor angiogenesis and proliferation signaling. The major targets include: receptor tyrosine kinase vascular endothelial growth factor receptors (VEGFR) 1 to 3, epidermal growth factor receptor (EGFR), fibroblast growth factor receptors (FGFR) 1 to 4, platelet-derived growth factor receptors (PDGFR) alpha and beta, and stem cell factor receptors (SCFR) 7, 8 and 9. One phase 2 clinical trial shows that anlotinib can improve progression-free survival with a potential benefit in overall survival (Han B, et al., Br J Cancer. 2018;118(5):654-661). One multicenter, double-blind, phase 3 randomized clinical trial shows that anlotinib results in prolonged overall survival and progression-free survival in Chinese patients, and this finding indicates that anlotinib is well tolerated, and is a potential third-line or further treatment medicament for patients with advanced NSCLC (Han B, et al., JAMA Oncol. 2018 Nov; 4(11):1569-1575).

The literature WO2008112407 discloses in Example 24 a quinoline derivative tyrosine kinase inhibitor 1-[[[4-(4-fluoro-2-methyl-1H-indol-5-yl)oxy-6-methoxyquinolin-7-yl]oxy]methyl]cyclopropylamine, and a preparation method thereof, the structural formula of which is represented by Formula I:

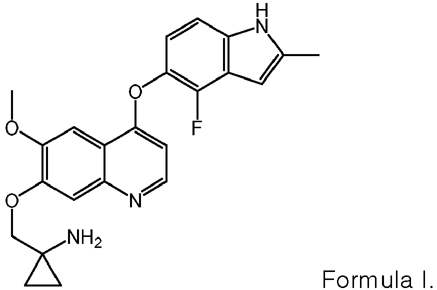

Tyvyt^{®} (sintilimab injection, Sintilimab, IBI308, IBI-308) is a human immunoglobulin G4 (IgG4) monoclonal antibody that can specifically bind to the PD-1 molecule on the surface of T cells, thereby blocking the PD-1/programmed death ligand 1 (Programmed Cell Death-1 Ligand-1, PD-L1) pathway which leads to tumor immune tolerance, reactivating the antitumor activity of lymphocytes, so as to achieve the goal of treating tumors. The sequence and structure of sintilimab can be found in antibody D of CN108473977A. On December 27, 2018, PD-1 antibody drug "sintilimab injection" from Innovent Biologics was officially approved by National Medical Products Administration (NMPA) of China for use in the treatment of relapsed or refractory classical Hodgkin's lymphoma at least received a second-line systemic chemotherapy.

The biggest challenge that previous researchers encounter in the process of tumor immunotherapy is the poor efficacy due to tumor immune tolerance and escape. Therefore, there is important theoretical significance and application value to break the well-established immune tolerance of body to tumor cells through the combined use based on small molecule anti-tumor compounds and anti-PD-1/PD-L1 antibodies.

### SUMMARY OF THE INVENTION

The present application provides a drug combination comprising a tyrosine kinase inhibitor and an isolated PD-1 antibody or antigen-binding fragment thereof. In some embodiments, the tyrosine kinase inhibitor is an EGFR inhibitor, a VEGFR inhibitor, a PDGFR inhibitor, and/or an FGFR inhibitor. In some embodiments, the tyrosine kinase inhibitor is a compound of Formula I above, or a pharmaceutically acceptable salt (e.g., hydrochloride salt) thereof. In some embodiments, the PD-1 antibody is selected from any one or more of toripalimab (JS-001), sintilimab (IBI308), camrelizumab, tislelizumab (BGB-A317), CS1003, HLX-10, AK103 (HX008), AK104, geptanolimab (GB226), Lizumab (LZM009), BAT-1306, SCT-I10A, F520, SG001, GLS-010, PDR001, REGN2810, STI-A1110. In some embodiments, the drug combination comprises an EGFR inhibitor or a VEGFR inhibitor and sintilimab or an antigen-binding fragment thereof. In some embodiments, the drug combination comprises a compound of Formula I or a pharmaceutically acceptable salt thereof (e.g., a hydrochloride salt), and sintilimab or an antigen-binding fragment thereof.

The present application provides a drug combination comprising a tyrosine kinase inhibitor and a human PD-1 antibody or an antigen-binding fragment thereof, and the human PD-1 antibody comprises a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6.

In some embodiments, the tyrosine kinase inhibitor is the compound of Formula I above, or a pharmaceutically acceptable salt thereof. In some specific embodiments, the tyrosine kinase inhibitor is a hydrochloride salt of the compound of Formula I, i.e., anlotinib hydrochloride.

In some embodiments, the human PD-1 antibody comprises a light chain variable region of which the amino acid sequence is set forth in SEQ ID NO:7 and a heavy chain variable region of which the amino acid sequence is set forth in SEQ ID NO:8. In some embodiments, the human PD-1 antibody comprises a light chain as set forth in SEQ ID NO:9 and a heavy chain as set forth in SEQ ID NO:10. In some embodiments, the human PD-1 antibody comprises a light chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 7 and a heavy chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 8. In some embodiments, the human PD-1 antibody comprises a light chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 9 and a heavy chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 10.

In some embodiments, the human PD-1 antibody is sintilimab.

In some embodiments, the compound of Formula I may exist in the form of a pharmaceutically acceptable salt or a pharmaceutically acceptable formulation thereof, preferably a hydrochloride salt thereof, e.g., a dihydrochloride salt.

In some specific embodiments, the compound of Formula I is a hydrochloride salt of 1-[[[4-(4-fluoro-2-methyl-1H-indol-5-yl)oxy-6-methoxyquinolin-7-yl]oxy]methyl]cyclopropylamine, i.e., anlotinib hydrochloride.

In some embodiments, the drug combination comprises: the compound of Formula I, or a hydrochloride salt thereof (e.g., dihydrochloride salt); and sintilimab, or an antigen-binding fragment thereof.

In some embodiments, the present application provides a drug combination comprising the compound of Formula I or a hydrochloride salt (e.g., dihydrochloride salt) thereof, and sintilimab or an antigen-binding fragment thereof. In some embodiments, the present application provides a drug combination, which is a formulation suitable for administration within a single treatment cycle (e.g., a 21-day treatment cycle), comprising 200 mg of sintilimab or an antigen-binding fragment thereof and a pharmaceutical composition comprising the compound of Formula I or a hydrochloride salt (e.g., dihydrochloride salt) thereof, the pharmaceutical composition comprising the compound of Formula I or a hydrochloride salt thereof comprises 84 to 168 mg of the compound of Formula I or the hydrochloride salt thereof by weight of the compound of Formula I. In some embodiments, the application provides a drug combination comprising the compound of Formula I or a hydrochloride salt thereof (e.g., dihydrochloride salt) and sintilimab or an antigen-binding fragment thereof in a ratio of (42-84):100 by weight of anlotinib (i.e., the compound of Formula I) and sintilimab or the antigen-binding fragment thereof. In some embodiments, sintilimab and anlotinib or a hydrochloride salt thereof or a pharmaceutical composition comprising anlotinib or a hydrochloride salt thereof may be packaged separately or together. In some embodiments, anlotinib or a hydrochloride salt thereof or a pharmaceutical composition comprising anlotinib or a hydrochloride salt thereof can be packaged in multiple aliquots (e.g., 2 aliquots, 7 aliquots, 14 aliquots, 28 aliquots or more); sintilimab can be packaged in a single aliquot or in multiple aliquots (e.g., 2 aliquots, 4 aliquots, or more).

The present application also provides an anti-tumor use of a drug combination comprising a tyrosine kinase inhibitor and a human PD-1 antibody or an antigen-binding fragment thereof, and the human PD-1 antibody comprises a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6. Alternatively, the present application also provides a drug combination for anti-tumor comprising a tyrosine kinase inhibitor and a human PD-1 antibody or an antigen-binding fragment thereof, and the human PD-1 antibody comprises a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6. The present application also provides a drug combination for anti-tumor comprising a tyrosine kinase inhibitor and an isolated PD-1 antibody or an antigen-binding fragment thereof. Wherein, the tyrosine kinase inhibitor and PD-1 antibody are as defined above.

In some embodiments, the tyrosine kinase inhibitor is the compound of Formula I or a hydrochloride salt thereof.

In some embodiments, the human PD-1 antibody comprises a light chain variable region of which the amino acid sequence is set forth in SEQ ID NO:7 and a heavy chain variable region of which the amino acid sequence is set forth in SEQ ID NO:8. In some embodiments, the human PD-1 antibody comprises a light chain as set forth in SEQ ID NO:9 and a heavy chain as set forth in SEQ ID NO:10. In some embodiments, the human PD-1 antibody comprises a light chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 7 and a heavy chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 8. In some embodiments, the human PD-1 antibody comprises a light chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 9 and a heavy chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 10. In some embodiments, the human PD-1 antibody is sintilimab.

In some embodiments, the anti-tumor use is directed to endometrial cancer. In some embodiments, the endometrial cancer is relapsed endometrial cancer or advanced endometrial cancer. In some embodiments, the endometrial cancer is relapsed or advanced endometrial cancer at least received a first-line platinum-based systemic chemotherapy.

In some embodiments, the application provides a drug combination against endometrial cancer comprising a tyrosine kinase inhibitor and a human PD-1 antibody or an antigen-binding fragment thereof, and the human PD-1 antibody comprises a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6. In some embodiments, the application provides a drug combination against endometrial cancer comprising the compound of Formula I or a hydrochloride salt (e.g., dihydrochloride salt) thereof and a human PD-1 antibody or an antigen-binding fragment thereof, and the human PD-1 antibody comprises a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6. In some embodiments, the human PD-1 antibody comprises a light chain variable region of which the amino acid sequence is set forth in SEQ ID NO:7 and a heavy chain variable region of which the amino acid sequence is set forth in SEQ ID NO:8. In some embodiments, the human PD-1 antibody comprises a light chain as set forth in SEQ ID NO:9 and a heavy chain as set forth in SEQ ID NO:10. In some embodiments, the human PD-1 antibody comprises a light chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 7 and a heavy chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 8. In some embodiments, the human PD-1 antibody comprises a light chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 9 and a heavy chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 10.

Alternatively, the present application also provides a drug combination against endometrial cancer comprising a tyrosine kinase inhibitor and an isolated PD-1 antibody or an antigen-binding fragment thereof. Wherein, the tyrosine kinase inhibitor and PD-1 antibody are as defined above.

In some embodiments, the present application provides a drug combination against endometrial cancer comprising the compound of Formula I or a hydrochloride salt (e.g., dihydrochloride salt) thereof and sintilimab or an antigen-binding fragment thereof.

In some embodiments, the application provides a drug combination against endometrial cancer, which is a preparation suitable for administration within a single treatment cycle (e.g., a 21-day treatment cycle), comprising 200 mg of sintilimab or antigen-binding fragment thereof and a pharmaceutical composition comprising the compound of Formula I or a hydrochloride salt (e.g., dihydrochloride salt) thereof, the pharmaceutical composition comprising the compound of Formula I or a hydrochloride salt thereof comprises 84 to 168 mg of the compound of Formula I or the hydrochloride salt thereof by weight of the compound of Formula I. In some embodiments, the application provides a drug combination against endometrial cancer comprising the compound of Formula I or a hydrochloride salt thereof (e.g., dihydrochloride salt) and sintilimab or an antigen-binding fragment thereof in a ratio of (42-84):100 by weight of anlotinib (i.e., the compound of Formula I) and sintilimab or the antigen-binding fragment thereof. In some embodiments, sintilimab and anlotinib or a hydrochloride salt thereof or a pharmaceutical composition comprising anlotinib or a hydrochloride salt thereof may be packaged separately or together. In some embodiments, anlotinib or a hydrochloride salt thereof or a pharmaceutical composition comprising anlotinib or a hydrochloride salt thereof can be packaged in multiple aliquots (e.g., 2 aliquots, 7 aliquots, 14 aliquots, 28 aliquots or more); sintilimab can be packaged in a single aliquot or in multiple aliquots (e.g., 2 aliquots, 4 aliquots, or more).

The present application also provides a method of treating or preventing a subject having endometrial cancer, comprising administering to the subject a therapeutically effective amount of a tyrosine kinase inhibitor and a therapeutically effective amount of a human PD-1 antibody or an antigen-binding fragment thereof, and the human PD-1 antibody comprises a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6. In some embodiments, the tyrosine kinase inhibitor is the compound of Formula I or a hydrochloride salt thereof. In some embodiments, the human PD-1 antibody comprises a light chain variable region of which the amino acid sequence is set forth in SEQ ID NO:7 and a heavy chain variable region of which the amino acid sequence is set forth in SEQ ID NO:8. In some embodiments, the human PD-1 antibody comprises a light chain as set forth in SEQ ID NO:9 and a heavy chain as set forth in SEQ ID NO:10. In some embodiments, the human PD-1 antibody comprises a light chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 7 and a heavy chain variable region having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 8. In some embodiments, the human PD-1 antibody comprises a light chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 9 and a heavy chain having at least 80% (e.g., at least 85%, at least 90%, at least 91%, at least 92%, at least 93%, at least 94%, at least 95%, at least 96%, at least 97%, at least 98%, at least 99%) homology to the amino acid sequence set forth in SEQ ID NO: 10. In some embodiments, the human PD-1 antibody is sintilimab. In some aspects, the endometrial cancer is relapsed endometrial cancer or advanced endometrial cancer.

The present application also provides a method of treating or preventing a subject having endometrial cancer, comprising administering to the subject a therapeutically effective amount of a tyrosine kinase inhibitor and a therapeutically effective amount of an isolated PD-1 antibody or an antigen-binding fragment thereof. Wherein, the tyrosine kinase inhibitor and the PD-1 antibody are as defined above.

In some embodiments, the present application provides a method of treating or preventing a subject having endometrial cancer, comprising administering to the subject a therapeutically effective amount of the compound of Formula I or a hydrochloride salt (e.g., dihydrochloride salt) thereof and a therapeutically effective amount of sintilimab or an antigen-binding fragment thereof. In some embodiments, the application provides a method of treating or preventing a subject having endometrial cancer, comprising administering to the subject 200 mg of sintilimab or an antigen-binding fragment thereof and a pharmaceutical composition comprising the compound of Formula I or a hydrochloride salt (such as dihydrochloride salt) thereof in a single treatment cycle (e.g., a 21-day treatment cycle), and the pharmaceutical composition comprising the compound of Formula I or a hydrochloride salt thereof comprises 84 to 168 mg of the compound of Formula I or the hydrochloride salt thereof by weight of the compound of Formula I. In some embodiments, the compound of Formula I or a hydrochloride salt (e.g., dihydrochloride salt) thereof, and sintilimab or an antigen-binding fragment thereof may be administered alone (e.g., the two are administered separately in succession, alternatively, the two are administered separately at regular intervals) or in combination (e.g., the two are administered simultaneously or substantially simultaneously).

The present application also provides a combination therapy for treating or preventing a subject having endometrial cancer, comprising administering to the subject a therapeutically effective amount of a tyrosine kinase inhibitor alone and a therapeutically effective amount of human PD-1 antibody alone, and the human PD-1 antibody comprises a light chain and a heavy chain, wherein the light chain comprises light chain complementarity determining regions LCDR1, LCDR2 and LCDR3 which respectively consist of the amino sequence set forth in SEQ ID NO: 1, SEQ ID NO: 2 and SEQ ID NO: 3, and wherein the heavy chain comprises the heavy chain complementarity determining regions HCDR1, HCDR2 and HCDR3 which respectively consist of the amino acid sequences set forth in SEQ ID NO: 4, SEQ ID NO: 5 and SEQ ID NO: 6. In some embodiments, the endometrial cancer is relapsed endometrial cancer or advanced endometrial cancer.

The present application also provides a method of treating or preventing a subject having a cancer or tumor which is endometrial cancer, comprising: (i) measuring the level of PD-1 and/or PD-L1 in a sample of the subject, wherein the subject is positive for PD-1 and/or PD-L1, and (ii) administering to the subject a therapeutically effective amount of anti-PD-1 and/or PD-L1 antibody or an antigen-binding portion thereof.

The present application provides a method of treating or preventing a subject having a cancer or tumor. In certain embodiments, the subject is a patient diagnosed with endometrial cancer, e.g., a patient diagnosed with relapsed endometrial cancer, or a patient diagnosed with advanced endometrial cancer.

In some embodiments of the present application, the subject has previously received surgery, chemotherapy and/or radiation therapy. In some specific embodiments, the subject has relapsed disease progression after achieving complete response following surgery, chemotherapy and/or radiation therapy. In some embodiments, the subject fails to achieve complete response or fails to achieve partial response following surgery, chemotherapy and/or radiation therapy.

In some embodiments of the present application, the subject has not previously received systemic chemotherapy. In some embodiments, the subject has previously received surgery, radiation therapy, induction chemotherapy, and/or adjuvant chemotherapy, or the subject receives concurrent chemotherapy. In some specific embodiments, the subject has not previously received systemic chemotherapy, but has received surgery, radiation therapy, induction chemotherapy, and/or adjuvant chemotherapy or will receive concurrent chemotherapy. In some specific embodiments, the subject has relapsed disease progression after achieving complete response following surgery, radiation therapy, induction chemotherapy, concurrent chemotherapy and/or adjuvant chemotherapy. In some specific embodiments, the subject fails to achieve complete response or fails to achieve partial response after receiving surgery, radiation therapy, induction chemotherapy, concurrent chemotherapy and/or adjuvant chemotherapy. In some embodiments, cancer has metastasized after the subject receiving surgery, radiation therapy, induction chemotherapy, concurrent chemotherapy, and/or adjuvant chemotherapy.

In some embodiments of the present application, the drug combination is a fixed combination. In some embodiments, the fixed combination is in the form of a solid pharmaceutical composition or a liquid pharmaceutical composition.

In some embodiments of the present application, the drug combination is a non-fixed combination. In some embodiments, the human PD-1 antibody and the compound of Formula I in the non-fixed combination are each in the form of a pharmaceutical composition.

The present application also at least provides a pharmaceutical pack comprising individually packaged pharmaceutical compositions in separate containers, respectively, wherein a pharmaceutical composition comprising the compound of Formula I or a pharmaceutically acceptable salt thereof is comprised in one container, and a pharmaceutical composition comprising a human PD-1 antibody is comprised in a second container.

In some embodiments of the present application, the pharmaceutical composition comprises the compound of Formula I in an amount of 56-168 mg, e.g., 84-168 mg. In some embodiments, the pharmaceutical composition comprises the compound of Formula I in an amount selected from 56 mg, 70 mg, 84 mg, 112 mg, 140 mg, 168 mg, or within a range defined by any two of the above values. In some embodiments, the pharmaceutical composition comprises the compound of Formula I in an amount of 112 mg to 168 mg.

In some embodiments, the human PD-1 antibody is administered at a dose of 1 mg/kg, 2 mg/kg, 3 mg/kg, 5 mg/kg, 6 mg/kg, 9 mg/kg, 10 mg/kg of body weight.

In some embodiments, the human PD-1 antibody is administered in one or more flat doses capable of effectively treating cancer(s). In some embodiments, the flat dose is in a range of about 10 mg to about 1000 mg of human PD-1 antibody. In some embodiments, the flat dose is selected from about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, or about 1000 mg of human PD-1 antibody. In some embodiments, the flat dose is selected from about 200 mg of human PD-1 antibody.

In some embodiments, the administration of human PD-1 antibody is in a 2-week (14-day) or 3-week (21-day) cycle, preferably administering the human PD-1 antibody intravenously on the first day (D1) of each cycle. That is, the anti-PD-1 antibody is administered at a frequency of once every two weeks (q2w) or once every three weeks (q3w).

The present application also provides a unit preparation, wherein the unit preparation comprises a compound component, which is 6-12 mg of the compound of Formula I or a hydrochloride salt thereof, and an antibody component, which is 50-350 mg of human PD-1 antibody or an antigen-binding fragment thereof; wherein the compound component and the antibody component are packaged separately.

The application also provides a method of preventing or treating cancer or tumor, wherein one or more of the above-mentioned unit preparations are administered to a subject in need thereof. Preferably, the compound component and the antibody component in the unit preparation are each administered separately. Preferably, the cancer or tumor is endometrial cancer.

### Anlotinib pharmaceutical composition

In the present application, an anlotinib pharmaceutical composition is any pharmaceutical composition comprising anlotinib or anlotinib hydrochloride (i.e., the compound of Formula I or a hydrochloride salt thereof) as an active ingredient. In this application, unless otherwise indicated, the dose of the anlotinib pharmaceutical composition is calculated by weight of anlotinib comprised therein.

In some embodiments of the present application, the unit dose of the anlotinib pharmaceutical composition comprises 2 mg, 6 mg, 8 mg, 10 mg, or 12 mg of anlotinib.

In some embodiments of the present application, according to a treatment cycle of 2 weeks of administration and 1 week of withdrawal, the total dose of the anlotinib pharmaceutical composition administered in each treatment cycle comprises 84-168 mg. In some embodiments, the total dose of the anlotinib pharmaceutical composition comprises those selected from 84 mg, 112 mg, 140 mg, 168 mg, or a range defined by any two of the aforementioned values. In some embodiments, the total dose of the anlotinib pharmaceutical composition preferably comprises 112 mg to 168 mg.

In some embodiments of the present application, anlotinib: 12 mg/tablet, one tablet per day, orally taken half an hour before breakfast, administered from the first day to the fourteenth day (d1-d14), every 3 weeks as a treatment cycle .

### Anlotinib

As used herein, the chemical name for anlotinib (i.e., the compound of Formula I) is 1-[[[4-(4-fluoro-2-methyl-1H-indol-5-yl)oxy-6-methoxyquinolin-7-yl]oxy]methyl]cyclopropylamine, which has the following structural formula:

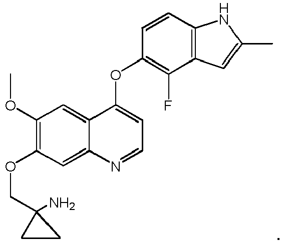

As used in the present application, anlotinib comprises non-salt form (e.g., free base) thereof, and also comprises a pharmaceutically acceptable salt thereof, and said non-salt form and salt are both included in the scope of the present application. For example, the pharmaceutically acceptable salt of anlotinib can be a hydrochloride salt or dihydrochloride salt. Unless otherwise stated, the doses of anlotinib or a salt thereof referred to in the present application are calculated based on anlotinib free base.

### Sintilimab

As used in this application, sintilimab (Sintilimab, IBI308, IBI-308) is an anti-PD-1 monoclonal antibody, of which the sequence and structure can be found in antibody D of CN108473977A. On December 27, 2018, PD-1 antibody drug "sintilimab injection" from Innovent Biologics was officially approved by National Medical Products Administration (NMPA) of China for use in the treatment of relapsed or refractory classical Hodgkin's lymphoma at least received a second-line systemic chemotherapy..

Full-length sequence of the heavy chain of sintilimab (SEQ ID NO: 10):

QVQLVQSGAEVKKPGSSVKVSCKASGGTFSSYAISWVRQAPGQGLEWMGLIIPMFDTAGY
AQKFQGRVAITVDESTSTAYMELSSLRSEDTAVYYCARAEHSSTGTFDYWGQGTLVTVSSA

STKGPSVFPLAPCSRSTSESTAALGCLVKDYFPEPVTVSWNSGALTSGVHTFPAVLQSSGL
YSLSSVVTVPSSSLGTKTYTCNVDHKPSNTKVDKRVESKYGPPCPPCPAPEFLGGPSVFLF
PPKPKDTLMISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEMTKNQVSL
TCLVKGFYPSDIAVEWESNGQPENNYKTTPPVLDSDGSFFLYSRLTVDKSRWQEGNVFSC
SVMHEALHNHYTQKSLSLSLGK.

Full-length sequence of the light chain of sintilimab (SEQ ID NO: 9):

DIQMTQSPSSVSASVGDRVTITCRASQGISSWLAWYQQKPGKAPKLLISAASSLQSGVPSR
FSGSGSGTDFTLTISSLQPEDFATYYCQQANHLPFTFGGGTKVEIKRTVAAPSVFIFPPSDE
QLKSGTASVVCLLNNFYPREAKVQWKVDNALQSGNSQESVTEQDSKDSTYSLSSTLTLSK
ADYEKHKVYACEVTHQGLSSPVTKSFNRGEC.

In some embodiments of the present application, IBI308 sintilimab 200 mg is continuously administered Q3W for 84 days (4 times) to achieve steady state, and the mean steady state trough concentration is expected to be about 26 µg/mL, which can sustainably maintain peripheral and target organ PD-1 receptor occupancy.

In some embodiments of the present application, sintilimab is administered at a single dose of 200 mg by intravenous infusion, once every 3 weeks, and every 3 weeks is a treatment cycle.

### Definitions and descriptions

Unless otherwise specified, the following terms used in the present application have the following meanings. A particular term should not be considered as being indefinite or unclear in the absence of a particular definition, but should be understood in accordance with the ordinary meaning of the term in the art. When a trade name appears in this application, it is intended to refer to its corresponding commercial product, composition or active ingredient.

As used herein, the term "antibody" refers to an antigen-binding protein having at least one antigen-binding domain. The antibodies and the fragment thereof in the present application may be an intact antibody or any fragments thereof. Thus, the antibody and the fragment thereof in the present application comprise a monoclonal antibody or a fragment thereof and an antibody variant or a fragment thereof, as well as an immunoconjugate. Examples of the antibody fragment comprise a Fab fragment, a Fab' fragment, a F(ab')₂ fragment, a Fv fragment, an isolated CDR region, a single chain Fv molecule (scFv), a Fd fragment and other antibody fragments known in the art. The antibody and the fragment thereof may also comprise a recombinant polypeptide, a fusion protein, and a bispecific antibody. The anti-PD-1 antibody/anti-PD-L1 antibody and the fragment thereof disclosed herein can be of IgG1, IgG2, IgG3, or IgG4 isotype.

The term "isotype" refers to the class of antibodies encoded by the heavy chain constant region gene. In one embodiment, the anti-PD-1/anti-PD-L1 antibody and the fragment thereof disclosed herein are of the IgG1 or IgG4 isotype. The anti-PD-1/anti-PD-L1 antibody and the fragment thereof in the present application can be derived from any species, including but not limited to mouse, rat, rabbit, primate, llama, and human. The PD-1/PD-L1 antibody and the fragment thereof may be a chimeric antibody, a humanized antibody or an intact human antibody. The term "humanized antibody" refers to an antibody in which the antigen-binding site is derived from a non-human species and the variable region framework is derived from a human immunoglobulin sequence. The humanized antibody may comprise substitutions in the framework regions such that the framework may not be an exact copy of the expressed human immunoglobulin or germline gene sequence. The term "human antibody" refers to an antibody in which all regions are encoded by human antibody genes.

An "isolated antibody" refers to an antibody that is substantially free of other antibodies having different antigenic specificities, e.g., an isolated antibody that specifically binds to PD-1/PD-L1 is substantially free of antibodies that specifically bind to antigens other than PD-1/PD-L1. However, an isolated antibody that specifically binds to PD-1/PD-L1 may have cross-reactivity with other antigens (such as PD-1/PD-L1 molecules from different species). Furthermore, an isolated antibody may be substantially free of other cellular materials and/or chemicals.

An "antigen-binding portion" (also referred to as an "antigen-binding fragment") of an antibody refers to one or more fragments of the antibody that retain the ability of specifically binding to an antigen to which the intact antibody binds.

As used herein, the term "derived" when used in reference to a molecule or polypeptide relative to a reference antibody or other binding proteins means a molecule or polypeptide capable of specifically binding to the same epitope as the reference antibody or other binding proteins.

As used herein, the term "EC50" refers to the effective concentration for 50% maximal response of an antibody. As used herein, the term "IC50" refers to the inhibitory concentration for 50% maximal response of an antibody. Both EC50 and IC50 can be measured by ELISA or FACS analysis or any other methods known in the art.

The term "treatment" generally refers to an operation to obtain a desired pharmacological and/or physiological effect. The effect may be prophylactic in terms of complete or partial prevention of the disease or its symptoms; and/or may be therapeutic in terms of partial or complete stabilization or cure of the disease and/or side effects due to the disease. "Treatment" as used herein encompasses any treatment of patient's diseases, including: (a) prevention of diseases or symptoms occurring in a patient who is susceptible to the diseases or symptoms but not diagnosed with the diseases; (b) suppression of symptoms of diseases, i.e. preventing the development thereof; or (c) alleviation of symptoms of diseases, i.e. causing the diseases or symptoms to subside.

As used herein, the term "systemic treatment" refers to a treatment in which a drug substance is transported through the bloodstream to reach and affect cells throughout the body.

As used herein, the term "systemic chemotherapy" refers to the whole-body chemotherapy excluding chemotherapy performed for locally advanced disease as part of multimodal therapy, wherein the chemotherapy performed for locally advanced disease includes induction chemotherapy, chemotherapy concurrent with radiation therapy, and adjuvant chemotherapy.

As used herein, the term "subject" refers to mammals such as rodents, felines, canines, and primates. Preferably, the subject according to the present application is human.

"Administration" means physically introducing a composition comprising a therapeutic agent to a subject using any one of a variety of methods and delivery systems known by the skilled in the art. Administration routes of immune checkpoint inhibitors (e.g., anti-PD-1 antibodies or anti-PD-L1 antibodies) include intravenous, intramuscular, subcutaneous, intraperitoneal, spinal or other parenteral administration routes, such as by injection or infusion. The phrase "parenteral administration" as used herein refers to the administration mode other than enteral and topical administration, usually by injection, and includes, but is not limited to, intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion, as well as electroporation *in vivo.* In certain embodiments, the immune checkpoint inhibitor (e.g., anti-PD-1 antibody or anti-PD-L1 antibody) is administered by a non-parenteral route, and in certain embodiments, it is administered orally. Other non-parenteral routes include topical, epidermal or mucosal administration route, e.g., intranasal, vaginal, rectal, sublingual, or topical. Administration can also be performed, for example, once, multiple times, and/or over one or more extended periods of time.

As used herein, an "adverse event" (AE) is any unfavorable and usually unintended or undesired sign (including abnormal laboratory findings), symptom, or disease associated with the application of a medical treatment. For example, an adverse event can be associated with activation of the immune system or expansion of immune system cells (e.g., T cells) in response to therapy. A medical treatment may have one or more associated AEs, and each AE may be of the same or different level of severity. Reference to a method capable of "modifying an adverse event" refers to a treatment regimen that reduces the incidence and/or severity of one or more AEs associated with the application of different treatment regimens.

As used herein, "dosing interval" refers to the amount of time that elapses among multiple doses of a formulation disclosed herein administered to a subject. The dosing interval can thus be indicated as a range.

The term "dosing frequency" as used herein refers to the frequency of administering a dose of a formulation disclosed herein within the given time. Dosing frequency can be indicated as the number of administrations per given time, e.g., once a week or once every two weeks.

Use of the term "flat dose" refers to the dose administered to a patient regardless of the patient's weight or body surface area (BSA). Therefore, the flat dose is specified as an absolute amount of an agent (e.g., anti-PD-1 antibody) rather than as a dose in mg/kg. For example, a 60 kg person and a 100 kg person will receive the same dose of antibody (e.g., 240 mg of anti-PD-1 antibody).

Use of the term "fixed dose" with respect to the compositions of the present application means that two or more different antibodies in a single composition are present in said composition in a specific (fixed) ratio to each other. In certain embodiments, the fixed dose is based on the weight (e.g., mg) of the antibody. In certain embodiments, the fixed dose is based on the concentration of the antibody (e.g., mg/mL). In certain embodiments, the ratio of a first antibody in mg to a second antibody in mg is at least about 1:1, about 1:2, about 1:3, about 1:4, about 1:5, about 1:1 6. About 1:7, about 1:8, about 1:9, about 1:10, about 1:15, about 1:20, about 1:30, about 1:40, about 1:50, about 1:60, about 1:70, about 1:80, about 1:90, about 1:100, about 1:120, about 1:140, about 1:160, about 1:180, about 1:200, about 200: 1, about 180:1, about 160:1, about 140:1, about 120:1, about 100:1, about 90:1, about 80:1, about 70:1, about 60:1, about 50:1, about 40:1, about 30:1, about 20:1, about 15:1, about 10:1, about 9:1, about 8:1, about 7:1, about 6:1, about 5:1, about 4:1, about 3:1, or about 2:1. For example, a 3:1 ratio of the first antibody to the second antibody may mean that the vial may contain about 240 mg of the first antibody and 80 mg of the second antibody, or about 3 mg/mL of the first antibody and 1 mg/mL of the second antibody.

The term "weight-based dose" as referred to herein refers to a dose administered to a patient calculated based on the patient's weight. For example, when a patient with a body weight of 60 kg requires 3 mg/kg of anti-PD-1 antibody and 1 mg/kg of anti-CTLA-4 antibody, one can draw appropriate amounts of anti-PD-1 antibody (i.e., 180 mg) and anti-CTLA-4 antibody (i.e., 60 mg) at one time from the fixed dose preparation of anti-PD-1 antibody and anti-CTLA-4 antibody in a ratio of 3:1. The term "immunotherapy" refers to the treatment of a subject afflicted with a disease or at risk of infection or suffering from relapse of a disease by certain methods that include inducing, enhancing, suppressing or otherwise altering an immune response. "Treatment" or "therapy" of a subject means any type of intervention or procedure performed on the subject, or administration of an active agent to the subject, for the purpose of reversing, alleviating, improving, inhibiting, slowing or preventing the onset, progression, development, severity, or relapse of a symptom, complication or condition, or disease-related biochemical markers.

As used herein, "PD1/PD-L1 positive" can be used interchangeably with "at least about 1% PD-1/PD-L1 expression". In one embodiment, PD-1/PD-L1 expression can be measured by any method known in the art. In another embodiment, PD-1/PD-L1 expression is measured by automated IHC. In certain embodiments, "PD-1/PD-L1 positive" refers to the presence of at least 100 cells expressing PD-1/PD-L1 on the cell surface.

"Programmed death receptor-1 (PD-1)" refers to an immunosuppressive receptor belonging to the CD28 family. PD-1 is expressed primarily on previously activated T cells *in vivo* and binds to two ligands PD-L1 and PD-L2. As used herein, the term "PD-1" includes human PD-1 (hPD-1), variants, congeners and species homologs of hPD-1, and analogs having at least one common epitope with hPD-1.

"Programmed death ligand-1 (PD-L1)" is one of two cell surface glycoprotein ligands directed to PD-1 (the other is PD-L2), which down-regulates T cell activation and cytokine secretion upon binding to PD-1.

"Subject" includes any human or non-human animal. The term "non-human animal" includes, but is not limited to, vertebrates such as non-human primates, sheep, dogs, and rodents such as mice, rats, and guinea pigs. In certain embodiments, the subject is human. The terms "individual", "subject", and "patient" are used interchangeably in certain contexts herein.

"Therapeutically effective amount" or "therapeutically effective dose" of a drug or therapeutic agent is any amount of the drug that, when used alone or in combination with another therapeutic agent, protects a subject from the onset of diseases or promotes regression of diseases, which is evidenced by a reduction in the severity of disease symptoms, an increase in the frequency and duration of disease symptom-free stages, or prevention of damage or disability caused by disease affliction. The ability of therapeutic agents to promote disease regression can be assessed using a variety of methods known by the skilled practitioner, such as in human subjects during clinical trials, in animal model systems for predicting efficacy in humans, or by measuring the activity of the agent in an *in vitro* assay.

As used herein, "subtherapeutic dose" refers to a dose of a therapeutic compound (e.g., antibody) that is lower than the usual or typical dose of the therapeutic compound when being administered alone for the treatment of a hyperproliferative disease (e.g., cancer).

As an example, "anticancer drug" promotes regression of cancer or prevents further tumor growth in a subject. In certain embodiments, a therapeutically effective amount of the drug promotes regression of the cancer to the point where the cancer is eliminated. "Promoting cancer regression" means that administering an effective amount of a drug, alone or in combination with an antitumor agent, results in a reduction in tumor growth or size, necrosis of the tumor, a reduction in the severity of at least one disease symptom, increase in the frequency and duration of disease symptom-free stages, or prevention of damage or disability caused by disease affliction. In addition, the terms "effective" and "effectiveness" in reference to a treatment include both pharmacological efficacy and physiological safety. Pharmacological efficacy refers to the ability of a drug to promote cancer regression in a patient. Physiological safety refers to the level of toxicity or other adverse physiological effects (adverse effects) resulting from drug administration at cellular, organ and/or organism level.

As an example for use in the treatment of tumors, a therapeutically effective amount of an anticancer drug can inhibit cell growth or tumor growth by at least about 10%, at least about 20%, at least about 40%, at least about 60%, or at least about 80% relative to an untreated subject, or in certain embodiments, relative to a patient treated with standard therapy of care. In other embodiments of the present application, tumor regression can be observed for a period of at least about 20 days, at least about 40 days, or at least about 60 days. Despite these ultimate measures of therapeutic effectiveness, the evaluation of immunotherapeutic drugs must also take into account "immune-related" response patterns.

"Immune-related" response pattern refers to a clinical response pattern frequently observed in cancer patients treated with immunotherapeutic agents that exert antitumor effects by inducing a cancer-specific immune response or by altering innate immune processes. This response pattern is characterized by a beneficial therapeutic effect following an initial increase in tumor burden or the appearance of new lesions, which would be classified as disease progression in the evaluation of traditional chemotherapeutics and would be synonymous with drug failure. Thus, proper evaluation of immunotherapeutic agents may require long-term monitoring of the effects of these agents on the target diseases.

A therapeutically effective amount of a drug includes a "prophylactically effective amount", which refers to any amount of a drug that inhibits the onset or relapse of cancer when being administered alone or in combination with an antitumor agent to a subject at risk of developing cancer (e.g., a subject with a premalignant condition) or a subject at risk of cancer relapse. In certain embodiments, a prophylactically effective amount completely prevents the onset or relapse of cancer. "Inhibiting" the onset or relapse of cancer means reducing the likelihood of the onset or relapse of cancer, or completely preventing the onset or relapse of cancer.

"Relapsed" cancer is one that regenerates at the initial site or a distant site after being responsive to an initial treatment (e.g., surgery). "Locally relapsed" cancer is one that occurs, after treatment, at the same location as the previously treated cancer.

"Advanced endometrial cancer" refers to endometrial cancer with local metastases (e.g., FIGO stage III) and distant metastases (e.g., FIGO stage IV).

"Unresectable" cancer is one that cannot be removed via surgery.

"Metastatic" cancer refers to one that spreads from one part of the body (e.g., lung) to another part of the body.

The application of alternatives (e.g., "or") shall be understood as referring to any one, two, or any combination of the alternatives. As used herein, the indefinite article "a" or "an" shall be understood as meaning "one or more" of any listed or enumerated components.

The terms "about", "approximately" or "essentially comprising" mean a value or composition within an acceptable error range of the particular value or composition determined by one of ordinary skill in the art, which will depend in part on how the value or composition is measured or determined, that is, the limits of the measurement system. For example, "about", "approximately" or "substantially comprising" can mean within 1 or more than 1 standard deviation, as practiced in the art. Alternatively, "about" or "substantially comprising" may refer to a range that differs by up to 10% or 20% (i.e., ±10% or ±20%) from the parameter or value modified by it. For example, about 3 mg can include any number between 2.7 mg and 3.3 mg (for 10%) or between 2.4 mg and 3.6 mg (for 20%). Furthermore, particularly with regard to biological systems or processes, the term may refer to up to an order of magnitude or up to 5 times of the value. When a particular value or composition is provided in this application and claims, unless stated otherwise, the meaning of "about" or "substantially comprising" should be assumed as being within an acceptable error range for that particular value or composition.

As used herein, the term "about once a week", "about once every two weeks", or any other similar dosing interval term refers to approximations. "About once a week" can include every 7 days ± 1 day, i.e., every 6 days to every 8 days. "About once every two weeks" can include every 14 days ± 3 days, i.e., every 11 days to every 17 days. Similar approximations apply to, for example, about once every 3 weeks, about once every 4 weeks, about once every 5 weeks, about once every 6 weeks, and about once every 12 weeks. In certain embodiments, a dosing interval of about once every 6 weeks or about once every 12 weeks means that the first dose may be administered on any day of the first week, followed by the administration of the second dose on any day of the sixth or twelfth week, respectively. In other embodiments, a dosing interval of about once every 6 weeks or about once every 12 weeks refers to administering the first dose on a particular day (e.g., Monday) of the first week and then administering the second dose on the same day (i.e., Monday) of the sixth or twelfth week, respectively. Similar principles apply to phrases including, but not limited to, "about once every 2 weeks", "about once a month", etc.

As described herein, any concentration range, percentage range, ratio range, or integer range should be understood as including any integer value within the recited range and, when appropriate, fractions thereof (such as one-tenth and one-percent of integers) being also included, unless otherwise indicated.

Unless otherwise stated, "about" or "approximately" in the present application refers to fluctuation within ±5%, preferably within ±2%, more preferably within ±1%, of the specified numerical range. For example, a pH value of about 5.5 means a pH of 5.5±5%, preferably a pH of 5.5±2%, more preferably a pH of 5.5±1%.

The term "pharmaceutically acceptable" is directed to those compounds, materials, compositions, and/or dosage forms which are, within the scope of sound medical judgment, suitable for use in contact with the tissues of human beings and animals without excessive toxicity, irritation, allergic response, or other problems or complications, commensurate with a reasonable benefit/risk ratio.

The term "pharmaceutically acceptable salt" includes salts formed by basic radicals and free acids and salts formed by acidic radicals and free bases, for example, hydrochloride, hydrobromide, nitrate, sulfate, phosphate, formate, acetate, trifluoroacetate, fumarate, oxalate, maleate, citrate, succinate, mesylate, benzenesulfonate, or *p*-methylbenzenesulfonate, preferably, hydrochloride, hydrobromide, sulfate, formate, acetate, trifluoroacetate, fumarate, maleate, mesylate, *p*-methylbenzenesulfonate, sodium salt, potassium salt, ammonium salt, and amino acid salt, etc. In the present application, when forming a pharmaceutically acceptable salt, the free acid and the basic radical are in a molar ratio of about 1:0.5 to 1:5, preferably 1:0.5, 1:1, 1:2, 1:3, 1:4, 1:5, 1:6, 1:7 or 1:8. In the present application, when forming a pharmaceutically acceptable salt, the free base and the acidic radical are in a molar ratio of about 1:0.5 to 1:5, preferably 1:0.5, 1:1, 1:2, 1:3, 1:4, 1:5, 1:6, 1:7 or 1:8.

The term "fixed combination" refers to administering the active components (for example, anti-PD-1 antibody or the compound of Formula I) to a subject simultaneously at a fixed total dose or in a fixed dose proportion, or in the form of a single entity, pharmaceutical composition or formulation.

The term "non-fixed combination" refers to administering two or more active components as independent entities (e.g., pharmaceutical compositions, formulations) to a subject simultaneously, concurrently, or sequentially and without a specific time limit, wherein the active ingredients administered to the subject reach the level of therapeutically effective amounts. An example, which can be listed, of the non-fixed combination is the cocktail therapy, for example, 3 or more active components are administered. In a non-fixed combination, each active component can be packaged, sold or administered as a completely independent pharmaceutical composition. The "non-fixed combination" also includes combined use between "fixed combinations", or a "fixed combination" and an independent entity of any one or more active components.

As used herein, "combined use" or "use in combination" means that two or more active substances may be administered to a subject together as a mixture, simultaneously as a separate formulation, or sequentially in any order as a separate formulation.

The term "pharmaceutical composition" refers to a mixture consisting of one or more of the active ingredients of the present application (e.g., an anti-PD-1 antibody or the compound of Formula I) or drug combinations thereof and a pharmaceutically acceptable excipient. The pharmaceutical composition is intended to facilitate the administration of the compound of the present application or the drug combination thereof to a subject.

The term "synergistic effect" refers to the situation where the effect (e.g., inhibition of colon cancer growth, or relief of colon cancer symptoms) resulted from two or more ingredients (e.g., anti-PD-1 antibody or the compound of Formula I) is greater than the simple addition of the effects of the ingredients administered alone. Unless otherwise stated, the terms "comprise", "comprises" and "comprising" or equivalents thereof herein are open-ended expressions and mean that other unspecified elements, components and steps may be encompassed in addition to those listed.

All patents, patent applications and other identified publications are expressly incorporated herein by reference for the purpose of description and disclosure. These publications are provided solely because they are disclosed prior to the filing date of the present application. All statements as to the dates of these documents or descriptions of the contents of these documents are based on the information available to the applicant and do not constitute any admission as to the correctness of the dates of these documents or the contents of these documents. Moreover, in any country, any reference to these publications herein does not constitute an admission that the publications form part of the common knowledge in the art.

### Mode of administration

The following content does not limit the mode of administration of the drug combination in the present application.

The components in the drug combination of the present application may each be formulated separately, or some or all of them may be formulated together. In one embodiment, the drug combination of the present application may be formulated as a pharmaceutical composition suitable for single or multiple administrations.

The components in the drug combination of the present application may each be administered separately, or some or all of them may be administered together. The components in the drug combination of the present application may be administered substantially at different times, or some or all of them may be administered substantially simultaneously.

The components in the drug combinations of the present application may each be administered independently, or some or all of them may be administered together, by various appropriate routes, including, but not limited to, oral or parenteral routes (by intravenous, intramuscular, topical, or subcutaneous routes). In some embodiments, the components of the drug combination of the present application may each be administered independently, or some or all of them may be administered together orally or by injection, e.g., intravenous or intraperitoneal injection.

The components in the drug combination of the present application may each independently, or some or all of them together be in a suitable dosage form, including, but not limited to, tablet, lozenge, pill, capsule (e.g., hard capsule, soft capsule, enteric-coated capsules, microcapsule), elixir, granule, syrup, injection (intramuscular, intravenous, intraperitoneal), powder, emulsion, suspension, solution, dispersion and dosage form of a sustained-release preparation for oral or non-oral administration.

The components in the drug combination of the present application may each independently, or some or all of them together comprise a pharmaceutically acceptable carrier and/or excipient.

The drug combination of the present application may also contain additional therapeutic agent. In one embodiment, the additional therapeutic agent may be a cancer therapeutic agent known in the art, preferably a lung cancer therapeutic agent.

In some specific embodiments, the present application investigates the efficacy of anlotinib hydrochloride and anti-PD-1 antibody alone or in combination against lung tumors. The experimental results surprisingly found that the combination use of anlotinib hydrochloride and anti-PD-1 antibody can have a significant synergistic effect, breaking the established immune tolerance to tumor cells in the body.

### EMBODIMENTS OF THE INVENTION

The present application is further described below with reference to specific examples, which are, however, only for illustration and do not limit the scope of the present application. Likewise, the present application is not limited to any particular preferred embodiment described herein. It should be appreciated by the skilled in the art that equivalent substitutions or corresponding improvements made to the technical features of the present application still fall within the scope of the present application. Unless otherwise stated, the reagents used in the following examples are all commercially available products, and the solutions can be prepared by conventional techniques in the art.

**Table 1 List of abbreviations**

| | | |
|---|---|---|
| AE | Adverse Event | Adverse Event |
| ALT | Alanine transaminase | Glutamic-pyruvic transaminase |
| AST | Aspartate amino transferase | Glutamic-oxalacetic transaminase |
| CR | Complete Response | Complete remission |
| Cr | Creatinine | Creatinine |
| CRF | Case Report Form | Case Report Form |
| DCR | Disease control rate | Disease control rate |
| DOR | Duration of response | Duration of response |
| ECG | Electrocardiogram | Electrocardiogram |
| ECOG | Eastern Cooperative Oncology Group | Eastern Cooperative Oncology Group |
| FDA | Food and Drug Administration | Food and Drug Administration |
| GCP | Good Clinical Practice | Good Clinical Practice |
| HBV | Hepatitis B virus | Hepatitis B virus |
| HCV | Hepatitis C virus | Hepatitis C virus |
| HIV | Human Immunodeficiency Virus | Human Immunodeficiency Virus |
| ICF | Informed Consent Form | Informed Consent Form |
| irAE | Immune-related Adverse Event | Immune-related Adverse Event |
| irRECIST | Immune-related Response Evaluation | Immune-related Response Evaluation Criteria In Solid Tumors |
| NYHA | New York Heart Association | New York Heart Association |
| ORR | Overall response rate | Objective response rate |
| OS | Overall Survival | Overall Survival |
| PD-1 | Programmed cell death 1 | Programmed death receptor-1 |
| PFS | Progression Free Survival | Progression Free Survival |
| PR | Partial Response | Partial Remission |
| RECIST | Response Evaluation Criteria In Solid Tumors | Response Evaluation Criteria In Solid Tumors |
| SAE | Severe Adverse Event | Severe Adverse Event |
| SD | Stable Disease | Stable Disease |
| TBIL | Total bilirubin | Total bilirubin |
| TMB | Tumor Mutation Burden | Tumor mutational burden |
| TTR | Time to response | Time to response |
| ULN | Upper limits of normal | Upper limits of normal |

### Example 1 Clinical study regimen of anlotinib hydrochloride combined with sintilimab in the treatment of relapsed or advanced endometrial cancer

This example discloses a clinical study regimen of anlotinib hydrochloride combined with sintilimab in the treatment of relapsed or advanced endometrial cancer. The main objective of the study was to evaluate ORR of IBI308 intravenous chemotherapy combined with anlotinib in the treatment of relapsed or advanced endometrial cancer after failure of first-line chemotherapy according to RECIST1.1 and irRECIST.

Primary endpoints: safety, ORR.

Secondary endpoints: DCR, PFS, OS.

ORR: Overall response rate, defined as the proportion of patients whose tumor volume is reduced to a pre-specified value and maintained for 4 weeks or more. Progression Free Survival (PFS): defined as the time from the first administration to objective tumor progression or death.

Overall Survival (OS): defined as the time starting from the first administration to death from any cause. Calculated in days, for subject lost to follow-up, the time of last follow-up was usually counted as the time of death.

Key enrollment criteria: >18 years old, female; histologically confirmed endometrial cancer; relapsed or advanced endometrial cancer at least received first-line platinum-based systemic chemotherapy; there are objectively measurable lesions according to RECIST 1.1 criteria, at least 1 target lesion.

Key Exclusion Criteria:
(1) Having components of carcinosarcoma (including malignant mixed Mullerian tumor, endometrial leiomyosarcoma and endometrial stromal sarcoma) pathologically;
(2) Having received the treatment of other antitumor drugs 4 weeks before the first administration;
(3) Having received major surgery within 28 days or being expected to require major surgery during the study period;
(4) Having received radiotherapy 21 days before administration (2 weeks for palliative radiotherapy for bone metastasis).
(5) Having received anti-PD1 antibody treatment previously;
(6) The patient still has unrelieved adverse reactions greater than grade 1 due to previous treatment (except for hair loss, greater than grade 2 for neurotoxicity);
(7) Immunosuppressant or systemic hormone therapy is being used to achieve the purpose of immunosuppression (prednisone or other equivalent hormone preparations with a dose >10mg/day), and is still being used 2 weeks before enrollment.
(8) Suffering from other malignant tumors in the past 5 years (except various in situ malignant tumors that have been fully treated, such as breast cancer, bladder cancer, cervical carcinoma in situ, skin basal cell carcinoma or squamous cell carcinoma).

### Doses for administration

Sintilimab: 200 mg by intravenous infusion, administered once every 3 weeks, with every 3 weeks as a treatment cycle.

Anlotinib: 12 mg/tablet, one tablet per day, orally taken half an hour before breakfast, d1-d14, with every 3 weeks as a treatment cycle. Patients were taken the study drugs until the treatment termination criteria specified by the regimen occurred.

Treatment termination criteria:
(1) Subject withdraws informed consent;
(2) The disease progression is shown by imaging examination;
(3) Due to the imaging false progression of PD-1, in addition to rapid and significant clinical progression, the first occurrence of disease progression needs to be confirmed 4-6 weeks later;
(4) The cumulative use of sintilimab has reached 2 years;
(5) Subject who is deemed as reaching CR by imaging examinations can consider withdrawal after 6 cycles of administration;
(6) Unable to tolerate toxicity;
(7) Poor compliance.

As a result, in clinical studies of relapsed or advanced endometrial cancer, anlotinib hydrochloride combined with sintilimab could be expected to show a certain therapeutic potential.

### Example 2 Treatment regimen

IBI308 (Sintilimab) 200mg/d, d1, intravenous chemotherapy, q3w.

The selection of the administration mode of sintilimab in this study was mainly based on the data of safety and exposure (concentration)-response (PD-1 receptor occupancy) relationship in the currently completed preliminary studies, combined with the preclinical in vitro/in vivo efficacy and related comparison data of similar drugs. On the premise that there was no significant variation in drug clearance characteristics of subjects, it was expected that continuous administration of 200mg IBI308 sintilimab Q3W for 84 days (4 times) could reach a steady state, and the mean steady state trough concentration was expected to be about 26µg/mL, which could sustainably maintain peripheral and target organ PD-1 receptor occupancy. After the first assessment of imaging progression by the investigators based on RECIST1.1 or irRECIST, if the clinical diseases of the subjects were stable, the investigators could communicate with the patients (relapsed or advanced endometrial cancer patients), and continue the current treatment after obtaining the informed consent from the patients. Imaging evaluation was performed again 4-6 weeks later for confirmation. If progression was confirmed again, treatment could be discontinued. The maximum duration of treatment with sintilimab was 24 months. During the study, the subject should continue to receive treatment until objective disease progression, intolerable toxicity, withdrawal of informed consent by the subjects, loss to follow-up or death occurred, or the investigators or sponsor decided to withdraw the subject from the trial.

Anlotinib hydrochloride: 12mg/tablet, qd, orally taken, d1-d14, q3w.

Until disease progression or intolerable adverse reactions occurred. If there was a missed dose during the medication and it was confirmed that the time for the next dose is shorter than 12 hours, the supplementary dose was not taken.

From the time of medication, tumor imaging evaluations were received at the second treatment cycle (±7 days) and the fourth week (±7 days), respectively, and then tumor imaging evaluations were performed once every 3 treatment cycles (±7 days) until the 10th cycle (i.e., the 7th and 10th cycles); after 10 treatment cycles, tumor imaging evaluations were performed once every 4 treatment cycles (±7 days). This study used the Simon's two-stage design to estimate the sample size. The Alpha value was 0.05 (one-sided), the Beta value was 0.2, and the test power was 0.8. It was found through this study that anlotinib hydrochloride combined with sintilimab could be expected to show a positive effect in the clinical treatment of patients with relapsed or advanced endometrial cancer.

### Example 3 Phase II clinical trial of anlotinib hydrochloride combined with sintilimab in the treatment of relapsed advanced endometrial cancer

### Methods

Patients who had received at least one platinum-based systemic chemotherapy, had Eastern Cooperative Oncology Group (ECOG) performance status of 0 or 1, and had disease measurable according to the Response Evaluation Criteria In Solid Tumors were considered as meeting the enrollment requirements. Sintilimab was administered intravenously (200 mg, q3w); anlotinib was administered orally (12 mg, qd, d1-14, 21 days per cycle). Treatment was continued until disease progression, death, or intolerable toxicity. The primary endpoint was overall response rate (ORR), and secondary endpoints were duration of response (DOR), disease control rate (DCR), progression-free survival (PFS), overall survival (OS), and safety.

From November 2019 to September 2020, a total of 21 patients were enrolled, with an average age of 56 years (range: 37-66 years), FIGO stage IA (about 23.8%), stage IB (about 9.5%), stage II (about 4.8%), stage IIIA (about 9.5%), stage IIIC (about 33.3%), stage IVB (about 19.0%). Among these patients, all 21 patients were evaluable.

### Results

Treatment evaluation showed that the incidences of complete response, partial response, stable disease, and disease progression were 9.6%, 52.4%, 19.0%, and 19.0%, respectively, with an ORR of 61.9% (95% CI: 38.4%-81.9%), DCR of 81.0% (95% CI: 58.1%-94.6%). Median PFS was not reached. Adverse events were predominantly grade 1 or 2. Grade 3 adverse events included ileus (4.8%), immune myocarditis (4.8%), immune peritonitis (4.8%), hand-foot syndrome (4.8%), neutropenia (4.8%), hypertension (4.8%); grade 4 adverse events included lymphocytosis (4.8%). There were neither unexpected safety signals nor treatment-related deaths.

It can be seen that anlotinib hydrochloride combined with sintilimab showed good toxicity properties and promising antitumor activity in patients with relapsed advanced endometrial cancer. Therefore, anlotinib hydrochloride combined with sintilimab can be expected to be clinically effective for the treatment of endometrial cancer patients.

According to the disclosure of the present application, although the compositions and methods of the present application have been described in terms of preferred embodiments, variations in the compositions and/or methods described herein as well as the steps or order of the steps of the methods can be made by the skilled in the art without departing from the concept, spirit and scope of the present application.

The disclosures of all documents cited herein are hereby incorporated by reference to the extent that they provide exemplary, procedural and other details supplementary to those described herein.

## Claims

1. A drug combination, comprising:
a) a human PD-1 antibody, comprising a light chain and a heavy chain, wherein the light chain comprises light chain complementarity determining regions LCDR1, LCDR2 and LCDR3 which respectively consist of the amino acid sequences set forth in SEQ ID NO: 1, SEQ ID NO: 2 and SEQ ID NO: 3, and wherein the heavy chain comprises heavy chain complementarity determining regions HCDR1, HCDR2 and HCDR3 which respectively consist of the amino acid sequences set forth in SEQ ID NO: 4, SEQ ID NO: 5 and SEQ ID NO: 6, and
b) a tyrosine kinase inhibitor, wherein the tyrosine kinase inhibitor is a compound of Formula I or a pharmaceutically acceptable salt thereof,

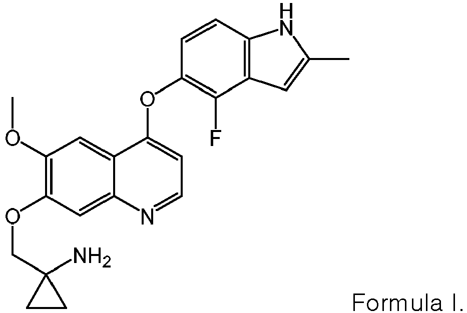

2. A drug combination, comprising a tyrosine kinase inhibitor and an isolated PD-1 antibody or an antigen-binding fragment thereof.

3. The drug combination according to claim 2, wherein the tyrosine kinase inhibitor is an EGFR inhibitor, a VEGFR inhibitor, a PDGFR inhibitor and/or an FGFR inhibitor.

4. The drug combination according to claim 2 or 3, wherein the tyrosine kinase inhibitor is a compound of Formula I or a pharmaceutically acceptable salt thereof.

5. The drug combination according to any one of claims 2-4, wherein the PD-1 antibody is selected from toripalimab, sintilimab, camrelizumab, tislelizumab, CS1003, HLX-10, AK103, AK104, geptanolimab, lizumab, BAT-1306, SCT-I10A, F520, SG001, GLS-010, PDR001, REGN2810 and/or STI-A1110.

6. The drug combination according to any one of claims 2-5, wherein the PD-1 antibody is sintilimab.

7. The drug combination according to any one of claims 1-6, wherein the pharmaceutically acceptable salt of the compound of Formula I is a hydrochloride salt, preferably dihydrochloride salt, of 1-[[[4-(4-fluoro-2-methyl-1H-indol-5-yl)oxy-6-methoxyquinolin-7-yl]oxy]methyl]cyclopropylamine.

8. The drug combination according to claim 1 or 7, wherein the human PD-1 antibody comprises a light chain variable region having at least 80% homology to the amino acid sequence set forth in SEQ ID NO: 7 and a heavy chain variable region having at least 80% homology to the amino acid sequence set forth in SEQ ID NO: 8.

9. The drug combination according to any one of claims 1 and 7-8, wherein the human PD-1 antibody comprises a light chain variable region having an amino acid sequence set forth in SEQ ID NO: 7 and a heavy chain variable region having an amino acid sequence set forth in SEQ ID NO: 8.

10. The drug combination according to any one of claims 1 and 7-9, wherein the human PD-1 antibody comprises a light chain having at least 80% homology to the amino acid sequence set forth in SEQ ID NO: 9 and a heavy chain having at least 80% homology to the amino acid sequence set forth in SEQ ID NO: 10.

11. The drug combination according to any one of claims 1 and 7-10, wherein the human PD-1 antibody comprises a light chain set forth in SEQ ID NO: 9 and a heavy chain set forth in SEQ ID NO: 10.

12. The drug combination according to any one of claims 1 and 7-11, wherein the human PD-1 antibody is sintilimab.

13. The drug combination according to claim 6 or 12, wherein the drug combination comprises the compound of Formula I or a hydrochloride salt thereof and sintilimab or an antigen-binding fragment thereof.

14. The drug combination according to claim 13, wherein in the drug combination, the compound of Formula I or the hydrochloride salt thereof and sintilimab or the antigen-binding fragment thereof are comprised in a ratio of (42-84):100 by weight of the compound of Formula I and sintilimab or the antigen-binding fragment thereof.

15. The drug combination according to claim 13, wherein the drug combination is a formulation suitable for administration within a single treatment cycle (e.g., a 21-day treatment cycle), comprising 200 mg of sintilimab or an antigen-binding fragment thereof and a pharmaceutical composition comprising the compound of Formula I or a hydrochloride salt thereof, and the pharmaceutical composition comprising the compound of Formula I or the hydrochloride salt thereof comprises 84 to 168 mg of the compound of Formula I or the hydrochloride salt thereof by weight of the compound of Formula I.

16. The drug combination according to any one of claims 13-15, wherein sintilimab and the compound of Formula I or the hydrochloride salt thereof are packaged separately or together.

17. The drug combination according to any one of claims 1-16, which is a non-fixed combination.

18. The drug combination according to claim 17, wherein the human PD-1 antibody and the compound of Formula I or the pharmaceutically acceptable salt thereof in the non-fixed combination are each in the form of pharmaceutical composition.

19. Use of the drug combination according to any one of claims 1-18 in the treatment or prevention of endometrial cancer.

20. The use according to claim 19, the drug combination is used for the treatment of relapsed endometrial cancer or advanced endometrial cancer.

21. The use according to claim 20, wherein the drug combination is used for the treatment of relapsed endometrial cancer or advanced endometrial cancer at least received a first-line platinum-based systemic chemotherapy.


SEQUENCE LISTING

<110>  CHIA TAI TIANQING PHARMACEUTICAL GROUP CO., LTD.
       INNOVENT BIOLOGICS (SUZHOU) CO., LTD.

<120>  DRUG COMBINATION OF QUINOLINE DERIVATIVE AND PD-1 MONOCLONAL ANTIBODY

<130>  October 30, 2019

<160>  10

<170>  SIPOSequenceListing 1.0

<210>  1
<211>  11
<212>  PRT
<213>  Artificial sequence

<220>
<223>  LCDR1

<400>  1
Arg Ala Ser Gln Gly Ile Ser Ser Trp Leu Ala
1               5                   10

<210>  2
<211>  8
<212>  PRT
<213>  Artificial sequence

<220>
<223>  LCDR2

<400>  2
Ser Ala Ala Ser Ser Leu Gln Ser
1               5

<210>  3
<211>  9
<212>  PRT
<213>  Artificial sequence

<220>
<223>  LCDR3

<400>  3
Gln Gln Ala Asn His Leu Pro Phe Thr
1               5

<210>  4
<211>  13
<212>  PRT
<213>  Artificial sequence

<220>
<223>  HCDR1

<400>  4
Lys Ala Ser Gly Gly Thr Phe Ser Ser Tyr Ala Ile Ser
1               5                   10

<210>  5
<211>  17

<212>  PRT
<213>  Artificial sequence

<220>
<223>  HCDR2

<400>  5
Leu Ile Ile Pro Met Phe Asp Thr Ala Gly Tyr Ala Gln Lys Phe Gln
1               5                   10                  15
Gly


<210>  6
<211>  13
<212>  PRT
<213>  Artificial sequence

<220>
<223>  HCDR3

<400>  6
Ala Arg Ala Glu His Ser Ser Thr Gly Thr Phe Asp Tyr
1               5                   10

<210>  7
<211>  107
<212>  PRT
<213>  Artificial sequence

<220>
<223>  a light chain variable region

<400>  7
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Ser Trp
            20                  25                  30
Leu Ala Trp Tyr Gln Gln Lys Pro Gly Lys Ala Pro Lys Leu Leu Ile
        35                  40                  45
Ser Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asn His Leu Pro Phe
                85                  90                  95
Thr Phe Gly Gly Gly Thr Lys Val Glu Ile Lys
            100                 105

<210>  8
<211>  120
<212>  PRT
<213>  Artificial sequence

<220>
<223>  a heavy chain variable region

<400>  8
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Ser Tyr
            20                  25                  30
Ala Ile Ser Trp Val Arg Gln Ala Pro Gly Gln Gly Leu Glu Trp Met
        35                  40                  45

Gly Leu Ile Ile Pro Met Phe Asp Thr Ala Gly Tyr Ala Gln Lys Phe
    50                  55                  60
Gln Gly Arg Val Ala Ile Thr Val Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ala Glu His Ser Ser Thr Gly Thr Phe Asp Tyr Trp Gly Gln
            100                 105                 110
Gly Thr Leu Val Thr Val Ser Ser
        115                 120

<210>  9
<211>  214
<212>  PRT
<213>  Artificial sequence

<220>
<223>  a light chain

<400>  9
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Ser Trp
            20                  25                  30
Leu Ala Trp Tyr Gln Gln Lys Pro Gly Lys Ala Pro Lys Leu Leu Ile
        35                  40                  45
Ser Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asn His Leu Pro Phe
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  10
<211>  447
<212>  PRT
<213>  Artificial sequence

<220>
<223>  a heavy chain

<400>  10
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Ser Tyr
            20                  25                  30
Ala Ile Ser Trp Val Arg Gln Ala Pro Gly Gln Gly Leu Glu Trp Met

35                  40                  45
Gly Leu Ile Ile Pro Met Phe Asp Thr Ala Gly Tyr Ala Gln Lys Phe
    50                  55                  60
Gln Gly Arg Val Ala Ile Thr Val Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ala Glu His Ser Ser Thr Gly Thr Phe Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Lys
        435                 440                 445